**Europäisches Patentamt**

**European Patent Office**

**Office européen des brevets**

(11) Publication number: **0 222 309**
**B1**

(12) **EUROPEAN PATENT SPECIFICATION**

(45) Date of publication of patent specification: **28.02.90**

(51) Int. Cl.⁵: **B 65 B 55/10, A 61 L 2/20**

(21) Application number: **86115310.4**

(22) Date of filing: **05.11.86**

(54) **Method for sanitizing packaging containers and materials, particularly for pharmaceutical and cosmetics use, and plant for implementing the method.**

(30) Priority: **11.11.85 IT 8415185**

(43) Date of publication of application:
**20.05.87 Bulletin 87/21**

(45) Publication of the grant of the patent:
**28.02.90 Bulletin 90/09**

(84) Designated Contracting States:
**AT BE CH DE ES FR GB GR IT LI LU NL SE**

(56) References cited:
**DE-A-1 492 369**
**DE-A-3 121 686**
**DE-A-3 324 939**
**GB-A- 760 733**
**US-A-3 549 528**

(73) Proprietor: **CONSIGLIO NAZIONALE DELLE RICERCHE**
**Piazzale Aldo Moro, 7**
**I-00198 Roma (IT)**
(73) Proprietor: **IDIM S.p.A. - ISTITUTO DERMATOLOGICO ITALIANO**
**Via Frigimelica, 20**
**35100 Padova (IT)**

(72) Inventor: **Bettero, Antonio**
**Via Leonardo da Vinci 12/A**
**I-35100 Padova (IT)**
Inventor: **Aversa, Graziana**
**Via Gobetti, 46**
**I-44100 Ferrara (IT)**
Inventor: **Cerini, Roberto**
**Via Ronchi, 4**
**I-35100 Padova (IT)**
Inventor: **Lucchiari, Mauro**
**Via Baccari, 41**
**I-45026 Lendinara (Rovigo) (IT)**
Inventor: **Giacomin, Renata**
**Via Valli, 25**
**I-35030 Galzignano (Padova) (IT)**
Inventor: **Scalabrin, Mario**
**Via Roma, 30**
**I-35040 Vescovana (Padova) (IT)**

Courier Press, Leamington Spa, England.

**EP 0 222 309 B1**

(72) Inventor: **Semenzato, Alessandra**
**Corso del Popolo, 21**
**I-35100 Padova (IT)**
Inventor: **Benassi, Carlo Alberto**
**Via Palestro, 4**
**I-35100 Padova (IT)**

(74) Representative: **Piovesana, Paolo et al**
**Corso del Popolo, 70**
**I-30172 Venezia-Mestre (IT)**

## Description

This invention relates to a method for dry sanitizing packaging containers and materials, particularly for pharmaceutical and cosmetics use, and a plant for implementing the method.

In the biomedical, food, pharmaceutical and cosmetics field, the various product containers have to be subjected to sanitizing processes before use, to ensure that the products remain preserved and unaltered for long time periods.

The known hot-sterilization method is not always applicable because certain packaging materials cannot be exposed to dry or moist heat. Because of this, alternative methods have been proposed comprising the use of electromagnetic radiation and chemical agents such as ethylene oxide, formaldehyde etc.

Although these systems have high sterilizing activity, they can modify the organoleptic characteristics of the various products, and can also produce side effects which are important from the safety aspect.

The bactericide properties of ozone are well known, but because of its toxic and reactive characteristics it has never been considered usable for sterilizing purposes other than for conditioning water, where it can be used practically without concentration limits because of its very rapid decomposition.

GB—A—760 733 refers to the use of ozone to sterilize surgical instruments, containers and products, which are not sensible to this gas.

DE—A1—3 324 939 refers to a method and a plant to sterilize surgical instruments in an ultrasonic bath, and teaches to introduce into the liquid an ozone stream at predetermined times. The nature of the materials to be treated excludes the need of controlling the ozone concentration during the treatment, and only foresees to neutralise it at the end of the same, to keep it under dangerous values, before discharging it into the atmosphere.

The object of the invention is to extend the field of application of ozone as a sanitizing agent, and to propose an industrial process and plant for sanitizing packaging material, particularly for pharmaceutical and cosmetics use.

This object is attained according to the invention by a method as described in claims 1—4.

The method according to the invention is implemented by a plant as described in claims 5—11.

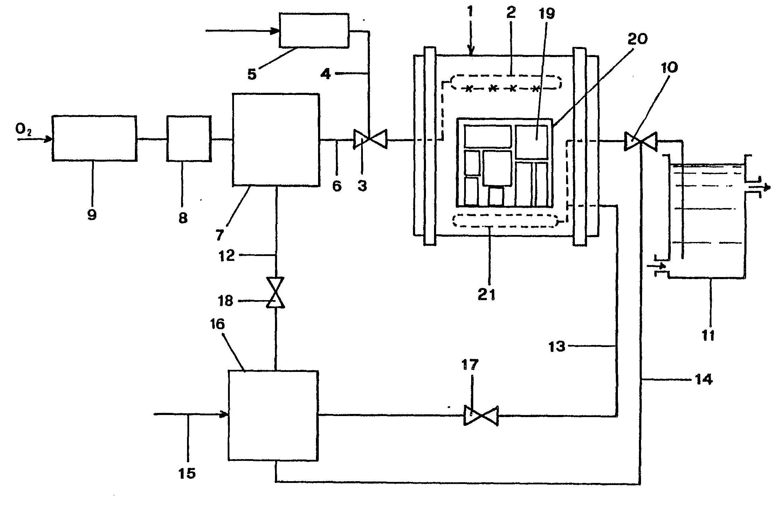

A preferred embodiment of the present invention is described in detail hereinafter by way of nonlimiting example with reference to the accompanying drawing, which diagrammatically illustrates a plant for implementing the method according to the invention.

As can be seen from the drawing, the plant according to the invention comprises essentially a sanitizing chamber 1, for example of cylindrical shape, with its inlet end located in the loading department for the containers to be sanitized, and its outlet end located in the packaging department.

In the top of the sanitizing chamber there is disposed a dispersing spreader 2 connected to the outside at the inlet end by way of a valve 3, to which two separate pipes are connected, the first pipe 4 being for feeding air made sterile by a filter 5, and the second pipe 6 for connection to an ozonising chamber 7, to which there is fed a stream of oxygen or oxygen-enriched air measured by a rotameter 9 and suitably dried by passing through a dryer 8.

The outflow from the sanitizing chamber 1 is connected by way of a discharge valve 10 to a container 11 in which the ozone leaving the sanitizing chamber 1 is decomposed in water. The ozone generator 7, the sanitizing chamber 1, the inlet of the decomposition device 11 and the surrounding atmosphere can be selectively connected by way of corresponding pipes 12, 13, 14 and 15 to an ozone sensor 16 of spectrophotometric type. In order to select the circuit to be measured, a change-over switch (not shown on the drawing) is provided together with shut-off valves 17 and 18 for closing the other circuits which are not to be measured.

The plant is constructed of materials which are resistent to the action of ozone under the operating conditions prevailing during industrial application.

The operation of the described plant is as follows: initially, the containers 19 to be sanitized are placed preferably randomly in stainless steel bins 20 in the chamber 1. This is then closed and put under slight vacuum. The chamber is then fed with a stream of ozone, which is drawn into it through the dispersing spreader 2 and leaves through an analogous coil structure 21 disposed in the bottom and directly connected to the discharge valve 10. Uniform diffusion of the gas takes place in this manner from the top downwards within the containers 19, independently of their shape. During this stage, the ozone concentration is continuously monitored spectrophotometrically by a quartz cell of the sensor 16.

Periodically, the ozone concentration of the surrounding atmosphere is also checked in order to prevent it exceeding predetermined safety values.

On termination of the sanitizing cycle, the chamber 1 is purged repeatedly with sterile air. The residual ozone is decomposed in water in the container 11, and eliminated. Finally, the chamber 1 is opened at its outlet end and the sanitized containers 19 are withdrawn directly into the packaging department.

From the foregoing it is apparent that the method according to the invention and the plant for implementing it have considerable advantages, in that they use ozone for the first time industrially for sanitizing containers, this being a gas which is easy to produce, with a short half life and thus much less risky than other chemical agents, it being also free from problems of environmental pollution by virtue of its decomposition in water, and, finally, being easy to control in spite of its toxicity by using simple safety measures such as hoods or other suction systems.

The following example will further clarify the invention.

A stainless steel sanitizing chamber was constructed having a capacity of about 15.000 l. During operation, it was put under slight vacuum (about 100 mm Hg). The continuous spectrophotometric monitoring was effected with a quartz cell having an optical path of 10 cm connected in series with the plant changeover switch. It enabled ozone to be analysed accurately and reproducibly from 0,01 ppm at a flow of 1 l/min.

In order to check the behaviour of the most common materials normally used for packaging cosmetics, the containers 19, suitably painted and/or xerographed, were treated with ozone at a concentration much higher than that used in an industrial sanitizing process, and their behaviour was observed after ageing tests.

More specifically, a significant sample of plastics containers normally used for cosmetics and withdrawn from the commercial production cycle was subjected to an ozone concentration of 30.000 ppm. After exposure for 5 to 10 minutes, the containers 19 were subjected to ageing tests at ambient temperature and at 40°C, both as such and after filling with a typical cosmetics product. They were then checked after six months and after one year.

The results, given in Table I, show that at the concentrations used, the ozone does not alter the structure and characteristics either of the containers or of the products contained in them.

TABLE I

Effects of ozone on packaging materials

| Containers | Materials | Products |
|---|---|---|
| Bottles | PVC | |
| | polyethylene | liquid soap |
| | polypropylene | |
| Bottles | polypropylene | semifluid emulsion |
| | polyethylene | |
| Tubes | aluminium | emulsions, |
| | epoxy resins | toothpastes |
| | melamine resins | |
| | polypropylene | |
| Jars | SAN (styrene-acrylonitrile) | thick emulsion |
| | polypropylene | |
| | polyethylene | |

Ozone concentration: 30.000 ppm;
Exposure time: 5 and 10 minutes;
Ageing: ambient temperature and 40°C;
Checking: after six months and one year.

*Modifications noted*
Containers: none
Products: none.

The following test was carried out to check the effect of ozone on various bacterial strains: initially, the ozone diffusion was checked within the containers 19 randomly placed in the sanitizing chamber 1 with the aid of chemical and bioindicators; with the sanitizing chamber under slight vacuum it was found that the ozone diffused uniformly within the containers 19, independently of their shape.

In order to evaluate the bactericidal activity of the ozone, tests were then carried out using various microbic species. For ease of handling, Durham tubes were placed randomly in baskets in the sanitizing chamber, the tubes containing suitable discs inoculated with suitable suspensions of various bacterial strains of different concentration and chemical indicators, for the double purpose of checking ozone diffusion and checking the actual time of initiation of the microbiological test. The time of contact of the ozone with the containers was kept constant at one or two hours, this time being considered sufficient for an industrial process, and the air or oxygen throughput was varied from 1 to 3 l/min and the ozone concentration at 50 to 500 ppm.

Table II shows the bacterial strains considered, and the results obtained for a concentration of 100 ppm.

TABLE II
Sanitization levels obtained after treatment with
100 ppm of ozone
(concentration of each species $10^6$ CFU/ml)

| Species | Treatment duration (minutes) | | | | | |
|---|---|---|---|---|---|---|
| | 60 | | | 120 | | |
| | Na | Nb | Is | Na | Nb | Is |
| St. Aureus | 50 | 2 | 0.04 | 50 | 1 | 0,02 |
| Str. Faecium | 50 | 2 | 0,04 | 50 | 1 | 0,02 |
| Ps. Aeruginosa | 50 | 1 | 0,02 | 50 | 0 | 0 |
| Ps. Delaphieldil | 50 | 0 | 0 | 50 | 0 | 0 |

Na = tested samples;
Nb = samples with positive growth;
Is = sanitization index (Nb/Na).

The data collected after a significant number of tests show that the vegetative forms are completely inactivated by the ozone.

## Claims

1. A method for dry sanitizing plastic packaging containers and materials, particularly for pharamaceutical, cosmetics and food use, characterized by:
   placing plastic containers in a sanitizing chamber,
   slightly evacuating said chamber until a pressure value is reached sufficient to ensure the uniform diffusion of ozone inside the containers,
   feeding an oxygen stream through an ozonizer to produce a stream of ozone,
   feeding a controlled quantity of the ozone stream into the sanitizing chamber,
   continuously spectroscopically monitoring ozone concentration in said chamber and controlling said concentration is not greater than predetermined safety values,
   maintaining the ozone in the sanitizing chamber for a predetermined time,
   purging said chamber with sterile air until a residual ozone concentration is obtained which is less than a predetermined safety value,
   decomposing the residual ozone by passing the stream of air and ozone leaving the sanitizing chamber through an ozone decomposition device, and
   opening the sanitizing chamber, removing the sanitized containers and directly transferring them to a packaging department.

2. A method as claimed in claim 1, characterised by starting with the sanitizing chamber under a vaccum of about 100 mm Hg.

3. A method as claimed in claim 1 characterised by feeding ozone into the sanitizing chamber through a disperser member which provides uniform ozone distribution within said chamber from the top to the bottom.

4. A method as claimed in claim 1, characterised by passing the air and ozone stream leaving the sanitizing chamber through water.

5. A plant for implementing the method claimed in one or more of claims 1 to 4, characterised by comprising:

an ozone generator (7) fed with a stream of dry oxygen,

a sanitizing chamber (1) fed by said generator (7),

means for purging the sanitizing chamber (1) with sterile air on termination of each sanitizing cycle, means (11) for decomposing the residual ozone on termination of purging,

means (16) for spectroscopically monitoring the ozone concentration in the generator (7), in the sanitizing chamber (1) and/or in the ozone decomposition means (11) and/or in the surrounding atmosphere.

6. A plant as claimed in claim 5, characterised in that the sanitizing chamber (1) is provided with an inlet opening and an outlet opening which are separate from each other and are in communication with the loading department for the containers and materials to be sanitized (19) and with the packaging department respectively.

7. A plant as claimed in claims 5 and 6, characterised in that the sanitizing chamber (1) is tunnel-shaped with its inlet and outlet opening provided at its ends.

8. A plant as claimed in claim 5, characterised in that the interior of the sanitizing chamber (1) is provided at its top with an ozone disperser (2) and at its bottom with an ozone collection coil (21), this latter disposed below the containers (19) to be sanitized and being of such dimensions as to generate, in combination with the dispenser (2), a uniform flow of ozone from the top downwards.

9. A plant as claimed in claim 5, characterised in that the oxygen concentration monitoring means (16) consist of a spectrophotometer with a quartz flow cell connected in series with the measuring circuit.

10. A plant as claimed in claim 5, characterised by comprising a multi-position switching device for selectively connecting the ozone concentration monitoring means (16) to the ozone generator (7), to the sanitizing chamber (1), to the decomposition means (11) and to the surrounding atmosphere.

11. A plant as claimed in claim 5, characterised in that the means (11) for decomposing the ozone leaving the sanitizing chamber (1) consist of a water container into which the outlet pipe from said sanitizing chamber (1) opens.

**Patentansprüche**

1. Verfahren zum Entkeimen von Verpackungsbehältern und Verpackungsmaterialien aus Kunststoff, insbesondere für die Verwendung im pharmazeutischen, kosmetischen und Nahrungsmittel-Bereich, gekennzeichnet durch:

Einsetzen der Kunstsoff-Behälter in ein Entkeimungskammer,

leichtes Evakuieren der Entkeimungskammer, bis ein Druck erreicht ist; der für eine gleichförmige Ozonverteilung in den Behältern ausreicht,

Zufuhr eines Sauerstoffstroms durch einen Ozonisator für die Erzeugung eines Ozonstromes,

Zufuhr einer gesteuerten Menge des Ozonstromes in die Entkeimungskammer,

kontinuierliche spektroskopische überwachung der Ozonkonzentration in der Entkeimungskammer und Sicherstellung, daß diese Konzentration nicht größer als ein vorbestimmter Sicherheitswert ist,

Verweilen des Ozons in der Entkeimungskammer für eine vorbestimmte Zeit,

Reinigen der Entkeimungskammer mit steriler Luft, bis ein Restozongehalt erreicht ist, der kleiner als der vorbestimmte Sicherheitswert ist,

Abbau des Restozons dadurch, daß der die Entkeimungskammer verlassende Luft- und Ozonstrom durch eine Ozonzersetzungseinrichtung geleitet wird,

öffnen der Entkeimungskammer, Entnahme der keimfrei gemachten Behälter und unmittelbare übergabe dieser Behälter an eine Verpackungsstation.

2. Verfahren nach Anspruch 1, dadurch gekennzeichnet, daß die Entkeimungskammer bei einem Vakuum von etwa 100 mm Hg in Betrieb gesetzt wird.

3. Verfahren nach Anspruch 1, dadurch gekennzeichnet, daß der ozon über ein Verteilorgan in die Entkeimungskammer geleitet wird, das in dieser vom Deckel bis zum Boden eine gleichmäßige Ozonverteilung vornimmt.

4. Verfahren nach Anspruch 1, dadurch gekennzeichnet, daß die Entkeimungskammer verlassende Luft- und Ozonstrom durch Wasser geleitet wird.

5. Anlage zur Durchführung des Verfahrens nach wenigstens einem der Ansprüche 1 bis 4, gekennzeichnet durch

einen mit einem Strom von trockenem Sauerstoff beschichten Ozonisator (7),

eine von dem Ozonisator (7) beschickte Entkeimungskammer (1),

Mittel zur Reinigung der Entkeimungskammer (1) mit steriler Luft am Ende jedes Entkeimungszyklus, eine Einrichtung (11) zum Zersetzen des Restozons am Ende der Reinigungsphase,

Mittel (16) zur spektroskopischen überwachung der Ozonkonzentration im Ozonisator (7), in der Entkeimungskammer (1) und/oder in der Einrichtung (11) zum Ozonabbau und/oder in der Umgebungsatmosphäre.

6. Anlage nach Anspruch 5, dadurch gekennzeichnet, daß die Entkeimungskammer (1) eine Beschickungsöffnung und eine Entnahmeöffnung aufweist, die voneinander getrennt sind und mit der Beschickungsstation für die zu entkeimenden Behälter und Materialien (19) bzw. mit der Verpackungsstation in Verbindung stehen.

7. Anlage nach Anspruch 5 und 6, dadurch gekennzeichnet, daß die Entkeimungskammer (1) die Form eines Tunnels mit den an den Enden ausgebildeten Beschickungs- und Entnahmeöffnungen hat.

8. Anlage nach Anspruch 5, dadurch gekennzeichnet, daß im oberen Bereich der Entkeimungskammer (1) ein Ozonverteiler (2) und im unteren Bereich eine Ozonsammelspule (21) angeordnet sind, wobei letztere unterhalb der zu entkeimenden Behälter (19) liegt und solche Abmessungen hat, daß sie zusammen mit dem Verteiler (2) einen gleichförmigen Ozonfluß von oben nach unten erzeugt.

9. Anlage nach Anspruch 5, dadurch gekennzeichnet, daß die Mittel (16) zur überwachung der Ozonkonzentration ein Spektralphotometer mit einem Quarzdurchflußelement aufweist, welche in Reihe mit dem Meßschaltkreis verdunden sind.

10. Anlage nach Anspruch 5, gekennzeichnet durch eine Mehrstellen-Schalteinrichtung zur wahlweisen Vebindung der Mittel (16) zur überwachung der Ozonkonzentration mit dem Ozonisator (7), mit der Entkeimungskammer (1), mit der Einrichtung (11) zum Ozonabbau und mit der Umgebungsatmosphäre.

11. Anlage nach Anspruch 5, dadurch gekennzeichnet, daß die Einrichtung (11) zum Abbau des die Entkeimungskammer (1) verlassenden Ozons aus einem Wasserbehälter besteht, in den die Ausgangsleitung der Entkeimungskammer (1) mündet.

## Revendications

1. Un procédé pour désinfecter à sec des récipients et des matériaux de conditionnement en plastique, en particulier à usage pharmaceutique, cosmétique et alimentaire, caracatérisé en ce qu'il consiste à:

placer des récipients en plastique dans une chambre de désinfection,
faire légèrement le vide dans ladite chambre jusqu'à ce que soit atteinte une valeur de pressione suffisante pour assurer la diffusion uniforme d'ozone à l'intérieur des récipients,
envoyer un courant d'oxygène à travers un ozoniseur pour produire un courant d'ozone,
envoyer un quantité controlée du courant d'ozone dans la chambre de désinfection,
surveiller en continu par spectroscopie la concentration d'ozone dans ladite chambre et contrôler que ladite concentration n'est pas supérieure à une valeur de sécurité prédéterminée,
maintenir l'ozone dans la chambre de désinfection pendant une durée prédéterminée,
purger ladite chambre avec de l'air stérile jusqu'à ce que soit obtenue une concentration d'ozone résiduel inférieure à une valeur de sécurité prédéterminée
décomposer l'ozone résiduel en faisant passer le courant d'air et d'ozone, qui quitte la chambre de désinfection, à travers un dispositif de décomposition d'ozone, et
ouvrir la chambre de désinfection, retirer les récipients désinfectés et les transférer directement dans un service de conditionnement.

2. Un procédé conforme à la revendication 1, caractérisé en ce qu'il consiste à commencer avec une chambre de désinfection sous une pressione d'environ 100 mm Hg.

3. Un procédé conforme à la revendication 1, caractérisé en ce qu'il consiste à envoyer de l'ozone dans la chambre de désinfection à travers un élément disperseur qui réalise une distribution uniforme d'ozone à l'intérieur de ladite chambre de haut en bas.

4. Un procédé conforme à la revendication 1, caractérisé en ce qu'il consiste à faire passer le courant d'air et d'ozone, qui quitte la chambre de désinfection, à travers de l'eau.

5. Une installation pour mettre en oeuvre le procédé conforme à une ou plusieurs revendications 1 à 4, caractérisée ce ce qu'elle comprend:

une générateur d'ozone (7) alimenté par un courant d'oxygène sec,
une chambre de désinfection (1) alimentée par ledit générateur (7),
un moyen pour purger la chambre de désinfection (1) avec de l'air stérile à la fin de chaque cycle de désinfection,
un moyen (11) pour décomposer l'ozone résiduel à la fin de la purge,
un moyen (16) pour surveiller par spectroscopie la concentration d'ozone dans le générateur (7), dans la chambre de désinfection (1) et/ou dans le moyen de décomposition (11) et/ou dans l'atmosphère ambiante.

6. Une installation conforme à la revendication 5, caractérisée en ce que la chambre de désinfection (1) est munie d'une ouverture d'entrée et d'une ouverture de sortie qui sont séparées l'une de l'autre et qui sont en communication avec le service de remplissage des récipients et des matériaux à désinfecter (19) et avec le service de conditionnement, respectivement.

7. Une installation conforme aux revendication 5 et 6, caractérisée en ce que la chambre de désinfection (1) est en forme de tunnel, avec ses ouvertures d'entrée et de sortie prévues à ses extrémités.

8. Une installation conforme à la revendication 5, caractérisée en ce que l'intérieur de la chambre de désinfection (1) est muni, dans sa partie haute, d'un disperseur d'ozone (2) et, dans sa partie basse, d'un enroulement collecteur d'ozone (21), ce dernier étant disposé sous les récipients (19) à désinfecter et ayant des dimensions propres à générer, en association avec la distributeur (2), un flux uniforme d'ozone à partir du haut.

7

9. Une installation conforme à la revendication 5, caractérisée en ce que le moyen de surveillance de concentration d'oxygène (16) consiste en un spectrophotomètre avec un cellule d'écoulement à quartz connectée en série avec le circuit de mesure.

10. Une installation conforme à la revendication 5, caractérisée en ce qu'elle comprend un dispositif de commutation à plusieurs positions pour connecter sélectivement le moyen de surveillance de concentration d'ozone (16) au générateur d'ozone (7), à la chambre de désinfection (1), au moyen de décomposition (11) et à l'atmosphère ambiante.

11. Une installation conforme à la revendication 5, caractérisée en ce que le moyen (11) pour décomposer l'ozone restant dans la chambre de désinfection (1) consiste en un récipient d'eau dans lequel débouche le tuyau de sortie provenant de ladite chambre de désinfection (1).